# EUROPEAN PATENT SPECIFICATION

(11) **EP 3 158 079 B1**
(45) Date of publication and mention of the grant of the patent: **27.02.2019**
(21) Application number: 15736414.2
(22) Date of filing: 19.06.2015
(51) Int. Cl.: C12Q 1/04, C12Q 1/689, G01N 33/569

(54) **DETECTION OF A SOURCE OF PINK DISCOLORATION DEFECT IN A SAMPLE**
NACHWEIS DER URSACHE EINES ROSA VERFÄRBUNGSDEFEKTS IN EINER PROBE
DÉTECTION D'UNE SOURCE D'UN DÉFAUT DE ROSE DÉCOLORATION DANS UN ÉCHANTILLON

(30) Priority: 19.06.2014 GB 201410948
(43) Date of publication of application: 26.04.2017
(73) Proprietor: Agriculture and Food Development Authority (TEAGASC), Co. Carlow (IE)
(72) Inventor: COTTER, Paul, Glanmire, Co. Cork (IE); SHEEHAN, Diarmuid JJ., Ballneety, Co. Limerick (IE); QUIGLEY, Lisa, Palmerstown, Dublin 20 (IE)
(74) Representative: Cahill, Susanne
(86) International application number: PCT/EP2015/063831
(87) International publication number: WO 2015/193482

(56) References cited:
- DIMITRIOS GIAGKAS ET AL: "Development of an Antibody for Detection of Rhamnolipids Characterized as a Major Bacterial Virulence Factor", ANTIBODIES, vol. 2, no. 3, 28 August 2013 (2013-08-28) , pages 501-516, XP055217703, DOI: 10.3390/antib2030501
- SATORU IWAI ET AL: "Silica deposition and phenotypic changes to Thermus thermophilus cultivated in the presence of supersaturated silicia", THE ISME JOURNAL, vol. 4, no. 6, 11 March 2010 (2010-03-11), pages 809-816, XP055217747, ISSN: 1751-7362, DOI: 10.1038/ismej.2010.12
- Y. FUJINO ET AL: "Thermus thermophilus TMY isolated from silica scale taken from a geothermal power plant", JOURNAL OF APPLIED MICROBIOLOGY, vol. 0, no. 0, 10 September 2007 (2007-09-10), XP055217754, ISSN: 1364-5072, DOI: 10.1111/j.1365-2672.2007.03528.x
- LANGEVELD LPM ET AL: "Adherence, growth and release of bacteria in a tube heat exchanger for milk", NETHERLANDS MILK AND DAIRY JOURNAL, PUDOC. WAGENINGEN, NL, vol. 49, 1 January 1995 (1995-01-01), pages 207-220, XP008177667, ISSN: 0028-209X
- CARINI S ET AL: "la colorazione rosanel formaggio fontina - [Pink discoloration in Fontina cheese] (translated)", IL LATTE, TECNICHE NUOVE, MILAN, IT, vol. 4, no. 2, 1 February 1979 (1979-02-01), pages 914-920, XP008177664, ISSN: 0392-6060
- T. OSHIMA ET AL: "Description of Thermus thermophilus (Yoshida and Oshima) comb. nov., a Nonsporulating Thermophilic Bacterium from a Japanese Thermal Spa", INTERNATIONAL JOURNAL OF SYSTEMATIC BACTERIOLOGY, vol. 24, no. 1, 1 January 1974 (1974-01-01), pages 102-112, XP055218133, ISSN: 0020-7713, DOI: 10.1099/00207713-24-1-102
- ANKE HENNE ET AL: "The genome sequence of the extreme thermophile Thermus thermophilus", NATURE BIOTECHNOLOGY, vol. 22, no. 5, 4 April 2004 (2004-04-04), pages 547-553, XP055218245, ISSN: 1087-0156, DOI: 10.1038/nbt956
- D. F. M. DALY ET AL: "Pink discolouration defect in commercial cheese: a review", DAIRY SCIENCE AND TECHNOLOGY, vol. 92, no. 5, 10 August 2012 (2012-08-10), pages 439-453, XP055218249, ISSN: 1958-5586, DOI: 10.1007/s13594-012-0079-0

## Description

### Background to the Invention

The pinking defect in cheese is a worldwide problem. It impacts on a range of ripened cheeses, including Swiss, Cheddar and Italian-type cheese as well as in ripened cheeses coloured with the food colouring, annatto. Its appearance can result in the downgrading or rejection of cheese and a consequential economic loss to producers. Pink discolouration defect can manifest in a number of ways depending on the cheese-type: at the surface of the cheese block either in patches or all over the block surface; as a uniform pink border occurring below the external surfaces of the cheese block conferring a pinked ring appearance; or sporadically distributed within the cheese block. While the cause of this defect is unknown, the topic has been the subject of much debate. It has been suggested that the pink defects are caused by physicochemical factors, though a microbial basis has also been proposed. Indeed, in the latter case, it has been noted that cheeses containing specific starter cultures, and strains of lactobacilli and propionic acid bacteria (PAB) in particular, are more likely to have a pink discolouration.

Daly et al. associate pink discoloration with certain strains of thermophilic lactobacilli and propionic acid bacteria.

### Statements of Invention

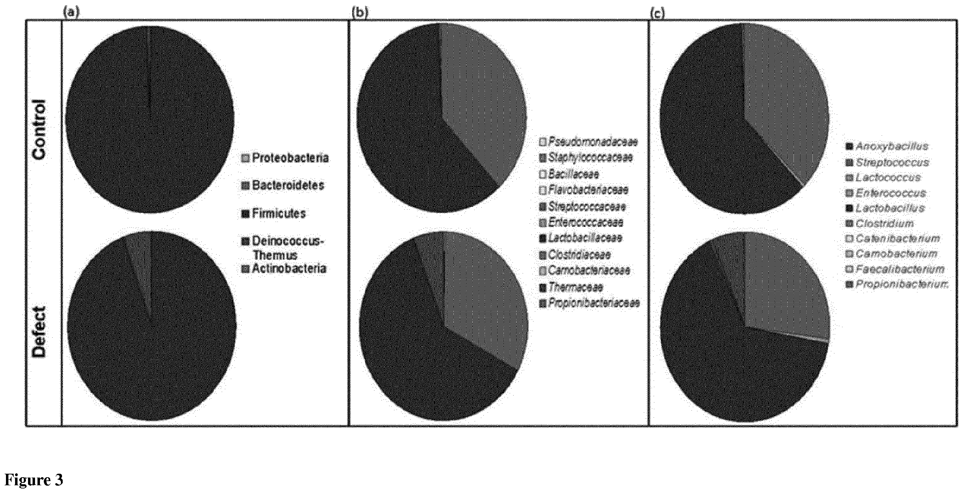

The Applicant has surprisingly discovered that the source of pink discoloration defect in cheese are bacteria that have heretofore not been associated with cheese production, *Thermus thermophilus.* High throughput DNA sequencing of the genome of control cheeses and cheeses having the pink coloration defect revealed the presence of *T. thermophilus* in defect cheeses at a significantly higher level than in control cheeses (Fig. 3c). In addition, following the production of three experimental continental-type cheeses spiked with *T. thermophilus* and unspiked control cheeses, it was apparent that the pink coloration defect occurred in spiked cheeses only.

Accordingly, in a first aspect, the invention broadly provides a method of determining the presence in a sample of a source of pink discoloration defect of cheese or a dairy product, comprising the steps of assaying the sample to detect the presence of a Thermus thermophilus bacteria in the sample, wherein the sample is an ingredient employed in cheese or dairy product manufacture or is obtained from a dairy product or cheese processing plant, and wherein detection of Thermus thermophilus bacteria in the sample indicates that the sample contains a source of pink discoloration defect of cheese or of a dairy product.

In particular, the invention relates to a method of determining the presence of a source of pink discolouration of cheese, however the invention may be applied to detecting the source of pink discolouration of other products, for example other dairy products such as, for example, milk, milk products, yoghurt, cream, butter, spreads, milk-derived protein powders and liquids including casein, whey protein, whole milk and skim milk powders.

In this specification, the term "pink discoloration defect" should be understood to mean the pink discoloration of dairy products or cheese, that in the case of cheese can occur at the surface of the cheese block in patches or all over the block surface, as a uniform pink border occurring below the external surfaces of the cheese block, or sporadically distributed within the cheese block.

In this specification, the term "*Thermus thermophilus*" or "*T. thermophilus*" should be understood to mean a bacteria of the genus *Thermus* and the species *thermophilus.* These are gram positive, extremely thermophilic, aerobic microorganisms, and are generally characterised by having a highly conserved region within the polymerase I gene.

An example of *Thermus thermophilus* is *T. thermophilus* DPC6866 and *T. thermophilus* HB27 (DSMZ Culture Collection, Germany)
In this specification, the term "detection of *Thermus thermophilus* bacteria in the sample" should typically be understood to mean detection of level of bacteria in the sample in excess of 10¹ cfu g⁻¹, for example at least 10² cfu g⁻¹ or 10³ cfu g⁻¹ as determined using the qPCR technique described below.

In the specification, the term "cheese" should be understood to mean any cheese, including hard, soft, semi-soft, or processed cheese products including processed cheese slices or cheese food products.

In this specification, the term "dairy product" should be understood to mean milk or any product made from milk, including cheese, yoghurt, butter, dairy spreads, and milk-derived protein powders and liquids or concentrates, such as for example casein, whey, whole milk and skim milk powders.

The sample may be obtained from a dairy or cheese processing plant, particularly a plant known to be producing cheese having the pink coloration defect. Generally, the sample is obtained from a surface of piece of machinery employed in the plant. Examples of plant or machinery employed in cheese or milk processing include milk vats, starter culture vats, pasteurisers. Typically, the sample is a swab taken from, for example, a surface of a machine The sample may also be an ingredient employed in cheese manufacture, for example, milk, starter culture, hot water, brine or any other ingredient that is used to make or process the cheese. The sample may also be an ingredient employed in dairy product manufacture, for example, milk, starter culture, water, hot water, brine, salt, sugar, or any other ingredient that is used to make or process dairy products.

Preferably, the sample is hot water.

In a second aspect, the invention provides a method of testing a dairy product or cheese manufacturing system for a risk of pink discoloration in the dairy product or cheese manufactured by the system, in which the dairy product or cheese manufacturing system comprises a cheese manufacturing plant and cheese manufacturing ingredients processed by the plant, a dairy product manufacturing plant and dairy product manufacturing ingredients processed by the plant, the method comprising the steps of assaying at least one sample obtained from the plant or ingredients to detect the presence of a *Thermus thermophilus* bacteria with the method of the invention detailed above, wherein detection of the *Thermus thermophilus* bacteria in at least one sample indicates a risk of pink discoloration in the dairy product or cheese manufactured by the system.

Suitably, the plurality of samples are obtained from the plant or ingredients and are selected from milk vat, starter culture vat, pasteuriser, steriliser, starter culture, water, hot water, brine, salt, sugar, CIP liquid. Preferably, the plurality of samples includes at least one sample from the cheese manufacturing plant and at least one sample from the cheese ingredients, at least one sample from the dairy product manufacturing plant and at least one sample from the dairy product ingredients.

In a further aspect, the invention provides a method of identifying an origin of pink coloration defect in a dairy product or cheese manufacturing system of the type comprising a cheese manufacturing plant and cheese manufacturing ingredients processed by the plant, or a dairy product manufacturing plant and dairy product manufacturing ingredients processed by the plant, the method comprising the steps of assaying samples obtained from a plurality of different sources selected from locations within the plant or ingredients to detect the presence of a *Thermus thermophilus* bacteria in the samples, according to the methods of the invention as detailed above, wherein detection of *Thermus thermophilus* bacteria in one of the samples indicates that the origin of the pink coloration defect is the source of the sample. Thus, if a sample obtained from hot water tests positive for *T. thermophilus,* this indicates that the hot water source is the origin of the pink discoloration defect. This will enable the plant operator to treat the hot water supply lines, storage vessels, and heating vessels, to eliminate the bacteria.

In the further aspect, the invention provides a method of modifying a cheese or dairy product manufacturing system of the type producing cheese or a dairy product having a pink discoloration defect, the method comprising the steps of identifying an origin of the pink coloration or defect according to a method of the invention, and modifying the cheese or diary product manufacturing system to remove or treat the origin of the pink discoloration defect. Where the origin of the pink coloration defect is colonisation of a location within the manufacturing plant with *Thermus thermophilus,* the surface is generally treated to remove the bacteria. Various methods of treatment to remove the bacteria will be apparent to a person skilled in the art, including treatment of the location with a sterilising liquid or gas.

Where the origin of the pink coloration defect is the presence of *Thermus thermophilus* in a manufacturing ingredient, the ingredient is generally replaced with an ingredient not containing *Thermus thermophilus.* The presence or otherwise of an ingredient to be used in cheese making may be determined by assaying the ingredient for presence of *Thermus thermophilus* bacteria, preferably quantitatively.

Preferably, the assay to detect the presence of *Thermus thermophilus* in the sample comprises polymerase chain reaction (PCR), preferably quantitative PCR. The details of PCR and qPCR will be well known to those skilled in the art, and involve amplification of a target sequence that is specific to the target bacteria, and not present in other bacteria known to be present in the sample. One example of a target sequence that is specific to *Thermus thermophilus* is a region of the polymerase I gene that can be amplified with PCR using the following primers:
Forward Primer (TpolFor): AGCCTCCTCCACGAGTTC [SEQUENCE ID NO: 1]
Reverse Primer: (TpolRev): GTAGGCGAGGAGCATGGGGT [SEQUENCE ID NO: 2]

Thus, in one preferred embodiment of the invention, the method employs PCR that is adapted to detect the presence of an amplicon that is unique to *Thermus thermophilus,* for example a target sequence that can be amplified using the primers of SEQUENCE ID NO'S 1 and 2, or a variant thereof that is specific to *Thermus thermophilus.*

Thus, in another aspect, described is a PCR kit, preferably a qPCR kit, for detecting the presence of *Thermus thermophilus* in a sample. The kit comprises a forward primer of SEQUENCE ID NO: 1 and a reverse primer of SEQUENCE ID NO: 2. Suitably, the kit comprises a control primer pair.

In another aspect, described is a quantitative PCR kit for specific quantitative detection of *Thermus thermophilus*, and comprising:
- a forward primer of SEQUENCE ID NO: 1 and a reverse primer of SEQUENCE ID NO:2; and optionally including one or more further reagents selected from buffer, dNTPs, thermostable hot-start DNA polymerase, and an appropriate dye (such as SYBR® Green).

Other methods for detecting the presence of *Thermus thermophilus* in a sample will be apparent to person skilled in the art, including use of antibodies that are specific to *Thermus thermophilus,* (doi:10.3390/antib2030501), for example a suitable ELISA or quantitative ELISA kit, or use of mass spectrophotometry.

It will be appreciated that other species of *Thermus* bacteria may be employed to detect the presence of a source of pink discolouration defect in a sample. Other *Thermus* species include *T. antranikianii*, *T. aquaticus, T. brockianus, T. caldophilus, T. filiformis*, *T. igniterrae, T. kawarayuensis, T. nonproteolyticus, T. oshimai, T. rehai, T. scotoductus, T. thermophilus, T. yunnanensi, T. sp. Manikaranii.* Methods for detecting these species of *Thermus* bacteria will be known by a person skilled in the art and from the literature in the field. (http://rd.springer.com/referenceworkentry/10.1007%2F0-387-30747-8 32. The Prokaryotes 2006, pp 797-812 The Genus Thermus and Relatives Milton S. Da Costa, Fred A. Rainey, M. Fernanda Nobre)

The invention also relates to the use of a kit for assaying a sample for the presence of a cause of pink discoloration defect, the kit comprising a diagnostic reagent for detecting the presence of *Thermus thermophilus* in a sample. Typically, the kit comprises means for quantitative detection of at least 10² or 10³ cfu.g⁻¹. Suitably the kit is a PCR, ideally a quantitative PCR kit. The kit comprises primer pairs adapted to quantitatively detect a target sequence in the *T. Thermophilus* DNA polymerase I gene that is unique to T. Thermophilus, i.e. a target sequence that can be amplified using a primer pair of SEQ ID NO's 1 and 2. Preferably, the PCR kit comprises a primer pair of SEQ ID NO: 1 and 2. As described herein, the kit comprises a probe adapted to specifically bind to a target sequence that is unique to *T. thermophilus.*

The disclosure relates to a kit for use in a method of the invention, for example for use in a method of determination of the presence of a source of pink discoloration in a sample.

### Brief Description of the Figures

**Figure 1****.** *Rarefaction curve of Shannon diversity indicating satisfactory coverage of all samples sequenced.*

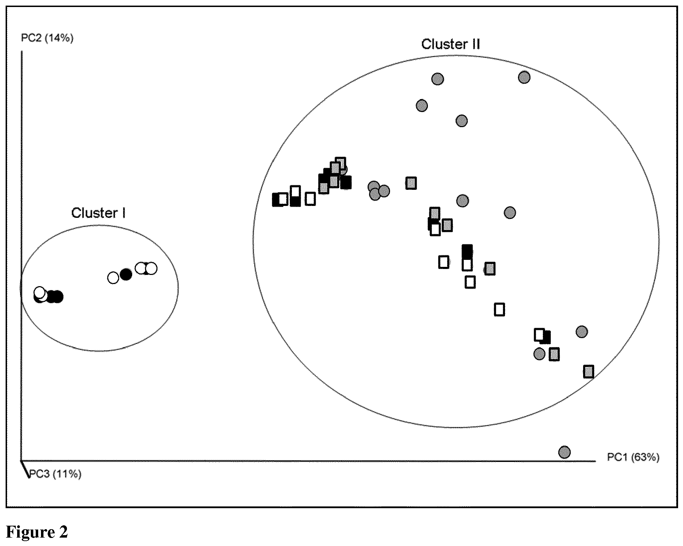

**Figure 2****.** *Principal Co-ordinate Analysis (PCoA) plot based on Weighted Unifrac, highlighting a split of the bacterial population into two clusters.* Two different cheese types manufactured using thermophillic cultures with cheese type 1 represented as dark red (control) and bright red (defect) and cheese type 2 represented in dark blue (control) and light blue (defect).
**Figure 3****.** *Bacterial composition of defect and control cheeses as determined by high throughput sequencing.* 16S rRNA sequences assigned according to MEGAN using the Silva database at the (a) phylum, (b) family and (c) genus levels with the three cheese types affected by the pink discolouration defect and controls populations.
**Figure 4****.** *Counts of ripening bacteria, Lactobacillus helveticus (Lh), Streptococcus thermophilus (St), propionic acid bacteria(PAB) and non-starter lactic acid bacteria (NSLAB) throughout ripening, 1d, 11d, 46d, 60d, 88d, 116d.*
**Figure 5****.** *Thermus thermophilus levels, as determined by qPCR, throughout manufacture.* M-inoculated milk, W-whey 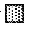 , C-curd. Experimental cheese 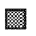 1, experimental cheese 2, experimental cheese 3 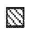 .
**Figure 6****.** *The effect of different treatments on cheese pH over ripening.* Control cheese 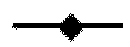 , experiment 1 cheese 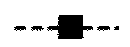 experiment 2 cheese 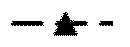 and experiment 3 cheese 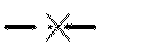 .
**Figure 7****.** *The effect of different experimental set-up on cheese* % *pH4.6 soluble nitrogen over ripening time.* Control cheese 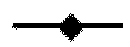 , experiment 1 cheese 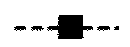 , experiment 2 cheese 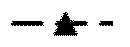 and experiment 3 cheese 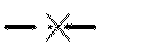 .
**Figure 8****.** *The effect of different experimental set-up on free amino acid levels after 116 days ripening.* Control cheese, experiment cheese 1, experiment cheese 2, experiment cheese 3.

### Detailed Description of the Invention

### Materials and Methods

### DNA extraction from cheeses

For nucleic acid extraction, 1 g of cheese from the defect or control cheese was combined with 9ml 2% tri-sodium citrate and homogenised before DNA was extracted using the PowerFood™ Microbial DNA Isolation kit (MoBio Laboratories Inc., USA) (Quigley et al., 2012a). As described previously (Quigley et al., 2012a), additional steps, whereby the homogenate was treated with 50 µg ml⁻¹ lysozyme and 100 U mutanolysin at 37°C for 1 hour followed with protein digestion by adding 250 µg ml⁻¹ proteinase K and incubating at 55°C for 1 hour, were added to the standard manufacturer's instructions.

### Generation of 16S rRNA amplicons for high throughput sequencing

DNA extracts were used as a template for PCR amplification of 16S rRNA tags (V4 region; 408 nt long) using universal 16S primers predicted to bind to 94.6% of all 16S genes i.e. the forward primer F1, 5'-AYTGGGYDTAAAGNG, SEQ ID 3 ((RDP's Pyrosequencing Pipeline: http://pyro.cme.msu.edu/pyro/help.jsp) and reverse primer V5, 5-CCGTCAATTYYTTTRAGTTT-3' SEQ ID 4 (Claesson et al., 2010). The primers incorporated the proprietary 19-mer sequences at the 5' end to allow emulsion-based clonal amplification for the 454-pyrosequencing system. Unique molecular identifier (MID) tags were incorporated between the adaptamer and the target-specific primer sequence, to allow identification of individual sequences from pooled amplicons. The PCR reaction contained 25 µl BioMix Red™ (Bioline Reagents Ltd., London, UK), 1 µl of each primer (10pmol), 5 µl DNA template and nuclease free H₂O to give a final reaction volume of 50 µl. PCR amplification was performed using a G-Storm thermal cycler (Gene Technologies, UK). The amplification programme consisted of an initial denaturation step at 94°C for 2 min, followed by 40 cycles; denaturation at 94°C for 1 min, annealing at 52°C for 1 min and extension at 72°C for 1 min. A final elongation step at 72°C for 2 mins was also included. Amplicons were cleaned using the AMPure XP purification system (Beckman Coulter, Takeley, United Kingdom). The quantity of DNA extracted by the different methods was assessed using the Quant-It™ Picogreen® dsDNA reagent (Invitrogen, USA) used in accordance with the manufacturer's instructions and a Nanodrop™ 3300 Fluorospectrometer (Thermo Fisher Scientific Inc, USA). The ND3300 excites in the presence of dsDNA bound with Picogreen® at 470nm and monitors emission at 525nm.

### High-throughput sequencing and bioinformatic analysis

The 16S rRNA V4 amplicons were sequenced on a 454 Genome Sequencer FLX platform (Roche Diagnostics Ltd, Burgess Hill, West Sussex, UK) according to 454 protocols. Read processing was performed using techniques implemented in the RDP pyrosequencing pipeline Sequences not passing the FLX quality controls were discarded, the 454 specific portion of the primer were trimmed, the raw sequences were sorted according to tag sequences and reads with low quality scores (quality scores below 40) and short length (less than 150 bp for the 16S rRNA V4 region) were removed as well as reads that did not have exact matches with the primer sequence. The QIIME suite of programs was used to align, chimera check, cluster and carry out phylogenetics on sequence reads, as well as, to measure microbial α-diversities and to plot rarefaction curves to determine if sequencing was carried out to sufficient depth β-diversities were calculated on the sequence reads based on Weighted Unifrac and principal coordinate analysis (PCoA) performed. KiNGviewer was used to visualise PCoA plots. Trimmed fasta sequences were assessed using BLAST against the SILVA version 100 database. The resulting BLAST output was parsed using MEGAN version 62.3.0. MEGAN assigns reads to NCBI taxonomies by employing the Lowest Common Ancestor algorithm which assigns each RNA-tag to the lowest common ancestor in the taxonomy from a subset of the best scoring matches in the BLAST result. Bit scores were used from within MEGAN for filtering the results prior to tree construction and summarisation (absolute cut-off: BLAST bit-score 86, relative cut-off: 10% of the top hit). The statistical significance of differences in proportions of microbial taxa was determined by the non-parametric Kruskal-Wallis test (Kruskal and Wallis, 1952) using Minitab® statistical package.

### Culturing of Thermus

### Culture-based Method

Castenholz TYE medium was chosen to selectively support the growth of strains from the genus *Thermus.* Castenholz TYE medium was prepared by mixing 5 parts 2X Castenholz salts with one part 1% TYE and 4 parts distilled water. Castenholz Salts, 2X contained 0.2 g nitrilotriacetic acid, 0.12g CaSO₄.2H₂0, 0.2g MgSO₄.H₂O, 0.016g NaCl, 0.21g KNO₃, 1.4g NaNO₃, 0.22g Na₂HPO₄, 2.0ml FeCl₃ solution (0.03%) and 2.0ml Nitsch's Trace elements {0.5ml H₂SO₄, 2.2g MnSO₄, 0.5g ZnSO₄.7H₂O, 0.5g H₃BO₃, 0.016g CuSO₄.5H₂O, 0.025g Na₂MoO₄.2H₂0, 0.046g CoCl₂.6H₂O distilled water 1L}, adjusted to a final volume of 1 L and final pH of 8.2. 1% TYE solution consisted of 10.0 g tryptone, 10.0 g yeast extract dissolved in 1 L distilled water. The final pH of Castenholz TYE medium should be 7.6. For preparation of the corresponding agar, 3% (w/v) bacteriological agar was added to the final solution. *Thermus* was isolated by enriching for 3 days at 70°C in Castenholz medium followed by isolation on Castenholz agar at 55°C for a further 3 days.

### PCR and qPCR-based detection of Thermus

A set of primers (TpolFor; 5'-AGCCTCCTCCACGAGTTC-3' and TpolRev; 5'-GTAGGCGAGGAGCATGGGGT-3') (SEQ ID 1 and 2) targeting a region specifically conserved within the polymerase 1 gene of *Thermus* were designed to facilitate PCR and qPCR-based detection of the genus. The theoretical specificity of these primers was tested using the oligo probe search tools in the BLAST classifier database. PCR amplification of the polymerase 1 gene using these primers was carried out under the following parameters: 95°C for 2 min initial denaturation, followed by 40 cycles of 94°Cx30 s, 63°Cx30 s, 72°Cx45 s, and a final elongation of 72°C for 2 min. The resultant products were visualised by agarose gel electrophoresis. Amplicons generated were cleaned using the Roche High Pure PCR clean-up kit and sequenced (Source Bioscience; Dublin, Ireland). The specificity of the primer pair was tested using DNA from a selection of cheese-associated Gram-positive and Gram-negative cultures i.e. *Streptococcus thermophilus* (Defined Starter Mix, TFP, France), *Lactobacillus helveticus* DPC6865, *Propionibacterium freudenreichii* DPC6451 and *Lactococcus lactis* HP as well as *Escherchia coli, Listeria monocytogenes* EGDe, *Salmonella typhimurium* LT2 and *Bifidobacterium longum* DPC15697.

To facilitate the quantification of *Thermus* by molecular means, a quantitative real-time (qPCR) protocol was designed. Genomic DNA was extracted from *Thermus thermophilus* HB27 (DSMZ Culture Collection, Germany) using the PowerFood Microbial DNA extraction kit (Cambio). A PCR product from within the polymerasel gene was generated using the genus-specific primers, as described above. Purified amplicons were cloned into the pCR®2.1-TOPO vector using the TOPO-TA cloning system (Invitrogen, Life Technologies, Carlsbad, California) in accordance with manufacturer's instructions. Following cloning, the complete vector was transformed into chemically competent TOP-10 *E. coli* cells (Invitrogen) and harvested on LB media containing 100 µg ml⁻¹ ampicillin. The accuracy of the cloned amplicon was confirmed by restriction analysis and DNA sequencing. QPCR standards were prepared following the linearization of plasmid DNA with *pst* restriction enzyme and quantification with the Nanodrop ND-1000 (Thermo Fisher Scientific Inc). A standard curve was then generated via a series of dilutions from 10² to 10⁸ copies µl⁻¹ DNA. The LightCycler 480 SYBR Green I Master kit (Roche Diagnostics GmbH, Mannheim, Germany) was used for quantification according to the manufacturer's instructions. Each PCR reaction contained 5µl Sybr green master mix (Roche), 1 µl of both forward and reverse primer (7.5 pmol), 2 µl of DNA and was made up to a final volume of 10 µl with nuclease free dsH₂O. The PCR conditions were as follows: an initial denaturation at 95°C for 10 min, followed by 45 cycles of denaturation at 95°C for 20 sec, annealing at 61°C for 15 sec and elongation 72°C for 20 sec. Assays were performed in triplicate. To facilitate quantification by qPCR, we applied the formula of Quigley et al., 2013, to convert from copies µl⁻¹ to cfu g⁻¹ of cheese.

### Cheese spiking studies

### Cheese manufacture and analysis

The starter cultures *S*. *thermophilus* (Defined Starter Mix, TPF, France) and *L. helveticus* DPC6865 were each grown overnight at 37°C in reconstituted low heat-skim milk powder, which had first been heat-treated at 90°C for 30 min. *Propionibacterium freudenreichii* DPC6451 was grown for 3 days at 30°C in sodium lactate broth.. *T. thermophilus* DPC6866, obtained from a cheese with a pink defect, was grown in Castenholz broth at 60°C with shaking for 36 hours. Cells were collected by centrifugation at 14,000 *g* for 20 min, washed once to remove trace media and resuspended in sterile water. Raw milk was obtained from Teagasc, Moorepark dairy herd, standardised, pasteurised at 72°C for 15 sec and pumped at 32°C into four individual cylindrical stainless steel vats with automated variable speed cutters and stirrers. This milk was employed to manufacture continental-type cheese at pilot-scale level in Moorepark Technology Ltd (Fermoy, Cork, Ireland). Details with respect to the manufacture of control and test cheeses can be found in Table 1. Enumeration of microbiological content, composition of cheeses and proteolysis were measured at various stages of ripening (Table 2). To enumerate specific bacterial components, cheese samples were aseptically removed, placed in a stomacher bag, diluted 1:10 with sterile tri-sodium citrate (2% w/v, Sigma) and homogenised in a Seward Stomacher® 400 Lab System (Seward Ltd., West Sussex, UK) for 2 min. Further dilutions were prepared as required. Viable *S. thermophilus* were enumerated on M17 agar (Oxoid) with 0.5% lactose (Oxoid) at 42°C for 3 days. *L. helveticus* were enumerated on MRS agar (Oxoid) adjusted to pH5.4 at 37°C for 3 days under anaerobic conditions. PAB levels were enumerated on sodium lactate agar containing 40 µg ml⁻¹ kanamycin (Sigma) at 30°C for 7 days under anaerobic conditions. Non-starter lactic acid bacteria (NSLAB) were enumerated on *Lactobacillus* Selective Agar (LBS; Difco) at 30°C for 5 days aerobically. *T. thermophilus* was monitored using qPCR methods. To facilitate this, DNA was extracted from milk, whey or 10 ml cheese homogenate using the PowerFood DNA isolation kit as described above. Grated samples from cheeses were analysed for salt (IDF, 1988), moisture (IDF, 1982) and protein (IDF, 1993) after 11 days of manufacture, pH (Standards, 1976) was measured throughout ripening. The levels of nitrogen soluble at pH 4.6 (pH 4.6SN) were measured as described by Sheehan et al. (2007). Free amino acid analysis was carried out on pH 4.6SN extract as described by Fenelon et al. (2000).

### Visual detection of pinking

Cheese rounds were examined visually throughout ripening for the formation of pink discolouration defect. Pink colour formation was quantified with a Chroma Meter using Hunter, ***L*, *a*, *b*** colour scale. The colour was measured using fresh sliced exposed cheese surface. The colour meter was standardised with a white standard plate (Y=88.31, x=0.3160, y=0.3226). Hunter ***a*** (redness) values were recorded.

### Statistical Analysis

A randomised complete block design that incorporated the four treatments and 3 blocks (replicate trials) was used for the analysis of response variables relating to the composition of cheeses, moisture, salt and protein, as well as starter bacteria, PAB, NSLAB, *T. thermophilus,* pH, pH4.6SN, FAA and apparent colour differences. Analysis of variance was carried out on data using the general linear model procedure of SAS (SAS Institute). The Tukey honestly significant difference test was used to determine the significance of difference between the means. The level of significance was determined at *p* <0.05.

### Results

### Compositional sequencing reveals higher proportions of the genus Thermus in cheeses with a pink defect

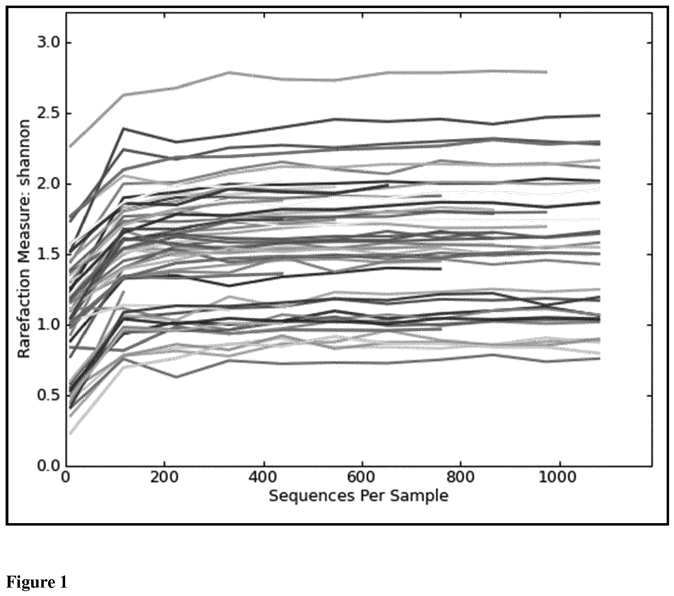

Compositional (16S rDNA) sequencing was performed on DNA extracted from control (n=9) and pink defect (n=9) samples of a commercially produced continental-type cheese. Sequencing coverage was satisfactory for all samples (SI Figure S1). Phylogenetic analysis established that the sequence reads corresponded to five different bacterial phyla (Figure 1a), *i.e.* Firmicutes, Proteobacteria, Bacteroides, Actinobacteria and Deinococcus-Thermus. Firmicutes and Deinococcus-Thermus dominated with less than 1% of assigned reads corresponding to other phyla. The proportions of Firmicutes present did not differ between control and defect samples. Reads corresponding to the phylum Deinococcus-Thermus were detected in defect-associated samples only (6%). When reads were assigned at the family level, eleven families were identified (Figure 1b). All reads from the phylum Deinococcus-Thermus were assigned to the family Thermaceae and, again, this was the only taxon for which significant differences were observed, i.e. 6% and 0% in defect and control, respectively. When these reads were assigned at genus level, 15 genera were identified (Figure 1c/SI Figure S2). Reads corresponding to Deinococcus-Thermus and Thermaceae were assigned to the genus Thermus and, again, this was the only taxonomic group for which there were significant differences (P = 0.002).

### Detection of Thermus in Cheese

Following the identification of reads assigned to the genus *Thermus* in samples of cheeses containing the pink discolouration defect, attempts were made to isolate this bacterium, which is not regarded as being a typical cheese-associated genus, from the defect cheeses. Castenholz medium was employed as it has previously been shown to support the growth of *Thermus* (Brock and Freeze, 1969) but, due to its minimal nutrient content, was unlikely to support the growth of other genera. An enrichment step, whereby cheese was homogenised in Castenholz medium and incubated at 70°C for 3 days, was employed to encourage the growth of *Thermus,* which are characterised by their highly thermophilic nature, and to prevent the growth of more moderately thermophilic cultures such as those within the starter culture population. A 3% agar was employed to allow incubation at high temperature (55°C) without rapid dehydration of the media. Use of this approach resulted in the successful isolation of *Thermus* from defect cheese.

Rapid, culture-independent PCR-based methods to detect *Thermus* were also developed. A primer pair was designed to selectively amplify the polymerase I gene of *Thermus* and assays with a broad variety of controls established the primers to be specific. To take full advantage of the specificity of these primers, a corresponding qPCR-based protocol was developed. Quantitative PCR analysis (of the cheeses used for 16S rDNA analysis) confirmed that *Thermus* was absent from the control cheeses and that defect cheeses contained on average 1.77 x 103 cfu g-1. Sequencing of PCR amplicons from defect cheeses and from *Thermus* strains isolated from these cheeses revealed that the species in question was *T. thermophilus.* A representative defect cheese isolate, T. thermophilus DPC6866, was employed in subsequent studies.

### Formation of "pinking" in spiked cheese

To establish definitively that *Thermus* is responsible for the formation of pink defects in cheese, a trial was carried out whereby cheese containing *T. thermophilus* was produced and the degree of pink development compared to that of a control cheese. For this, a continental-type cheese was manufactured. Trials were carried out in triplicate and in each instance four cheeses were produced. These included the control cheese, which contained no *T. thermophilus,* and three experimental cheeses, all of which contained *T. thermophilus* at 10⁶ cfu g⁻¹. Experiment 1 (exp 1) cheese contained *T. thermophilus* with starter cultures at normal levels (500 g *L. helveticus,* 250 g *S*. *thermophilus,* 4 g PAB). Experiment 2 (exp 2) cheese contained *T. thermophilus* with higher levels of *L. helveticus* (500 g). Finally, experiment 3 (exp 3) cheese contained *T. thermophilus* with higher levels of *L. helveticus* (500 g) and lower levels of *S*. *thermophilus* (250 g) (Table 1). The reasoning behind the varying levels of *L. helveticus* and *S*. *thermophilus* was due to the increased and decreased levels of these bacteria (respectively), as detected by the pyrosequencing data where *Thermus* was present.

### Starter, PAB and NSLAB viability during cheese ripening

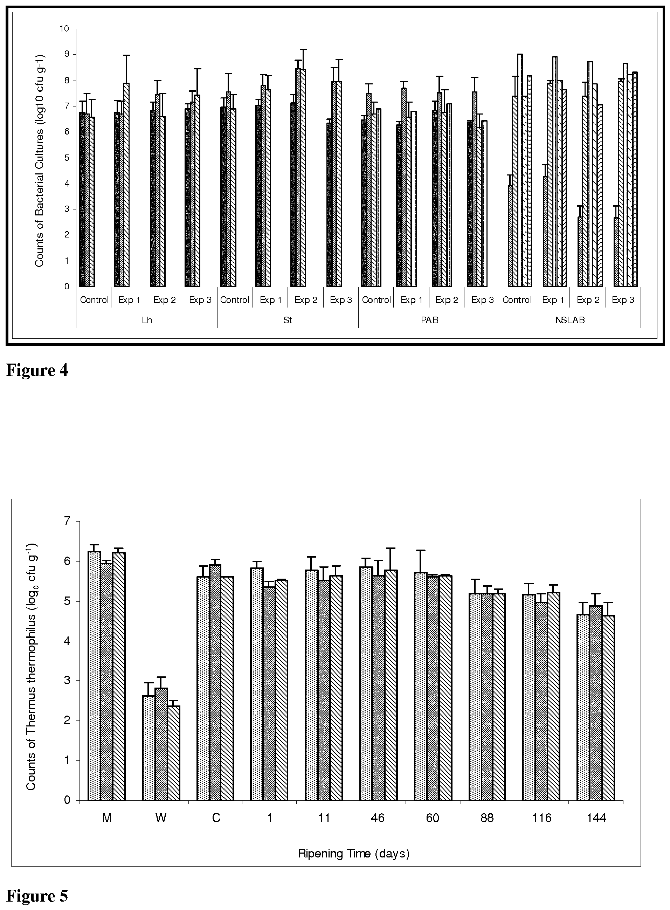

Mean viable cell numbers of *S*. *thermophilus* were determined to be 10⁷ cfu g⁻¹ at day 1 of ripening in control, exp 1 and exp 2 cheeses and at 10⁶ cfu g⁻¹ in exp 3 cheese, which correlates with levels of starter *S*. *thermophilus* inoculated into the cheese milk. There were significant increases (*p*=0.0063) in the numbers of S. *thermophilus* over time (Figure 4) but there were no significant differences between treatments. *L. helveticus* numbers were 1 x 10⁶ cfu g⁻¹ at 1 d ripening, in control and exp 1 cheese, while exp 2 and exp 3 cheese contained 5 x 10⁶ cfu g⁻¹, again reflecting the different levels of *L. helveticus* starter added (Figure 4). The changes observed in levels of *L. helveticus* during cheese production were not significant. Counts of PAB increased significantly until 46 d ripening (*p*<0.0001) (Figure 4), however they did not differ significantly between treatments. Viable NSLAB numbers increased significantly until the end of warm room ripening (Figure 4) (*p*<0.0001). We observed a significant difference in the levels of NSLAB between control cheese and exp 2 cheese (*p*=0.0438) and control cheese and exp 3 cheese at 60 d ripening (*p*=0.0225).

### Survival of Thermus thermophilus throughout cheese manufacture and ripening

*Thermus thermophilus* was inoculated with a view to obtaining >10⁴ cfu g⁻¹ in the three experimental cheeses. Using culture-independent qPCR, the levels of *T. thermophilus* present in the inoculated milk, lost in whey, and retained in curd, as well as throughout ripening were determined (Figure 5). *Thermus* was present at 10⁶ cfu ml⁻¹ in milk after 1 h inoculation (sampled prior to rennet addition). There was some loss of *T. thermophilus* in whey, i.e. 10² cfu ml⁻¹, however, considerable levels were retained within the curd (10⁵ cfu g⁻¹). Control cheeses, which were not spiked with *T. thermophilus,* were also assessed and were found not to contain *Thermus* (data not shown), establishing that no natural contamination, or cross-contamination, occurred during production. Slight numerical increases in the levels of *T. thermophilus* were noted during hot room ripening, however these were not significant. Following transfer to the cold room for continued ripening, we observed a slight decrease in the levels of *T. thermophilus* to 10⁴ cfu g⁻¹. This was consistent across all three experimental cheeses (Figure 5).

### Composition of cheeses

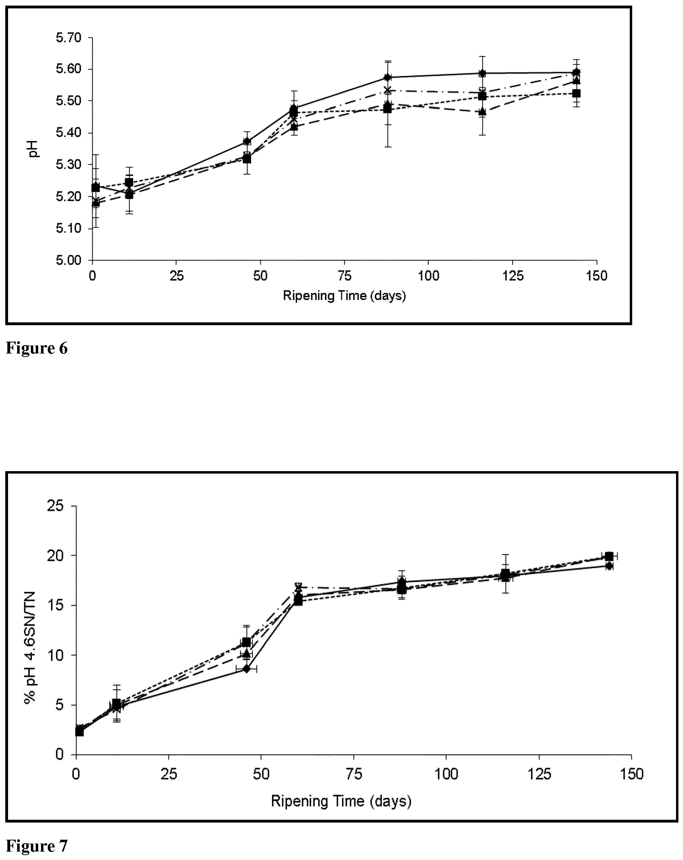

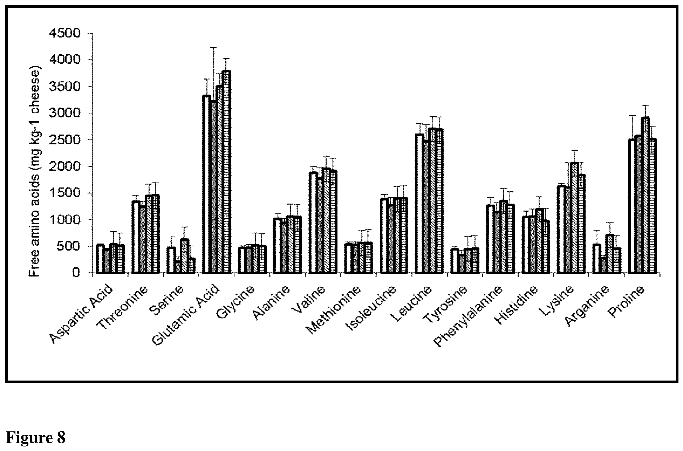

The gross composition of cheeses at 11 d ripening was assessed and is summarised in Table 3. All cheeses had statistically similar pH values, levels of moisture, salt and protein. The consistency of these results between cheeses and cheese trials indicate good repetition across each day of manufacture i.e. no significant differences were detected between these variables. Significant increases in pH (Figure 6), pH 4.6SN (soluble nitrogen) (Figure 7) and total FAA (*p*<0.0001 for all three parameters assessed) were observed throughout ripening. The concentrations of individual FAAs (mg kg⁻¹ of cheese) in all cheeses at 116 d of ripening are shown in Figure 8. The FAAs present at greatest concentrations in the cheeses at most ripening times were glutamic acid, valine, leucine, lysine and proline, and were in line with that expected in Swiss-type cheeses (Sheehan et al., 2008).

### Visual Examination for "pinking" formation in cheese

To quantify the formation of "pinking" in the cheese samples we applied a Chroma Meter using Hunter ***L*, *a*, *b*** colour scale throughout ripening. Hunter ***a*** values determine the level of redness (+) to greenness (-) (Wadhwani and McMahon, 2012). Changes in the ***a*** values are summarised in Table 4. The ***a*** reading is a negative value, establishing that the overall colour is in the green spectrum. However, throughout the centre of the experimental cheeses there is a shift towards a more positive value. These differences were first noted after 116 d ripening (*a* = -2.08, -1.91, -1.75, -1.73, for control, exp 1, exp 2 and exp 3 cheeses, respectively) and the intensity of this value and the formation of a pink hue developed further in exp 2 cheese at 144 d, (*a*=-2.38 - 1.95, -1.34 and -1.82 for control, exp 1, exp 2 and exp 3 cheeses, respectively). This further pinking of exp 2 cheese between 116 d and 144 d was statistically significant (*p*=0.0108). The exp 2 144 d values were also significantly less negative than those of the control (*p*=0.0009) and exp 1 cheeses (*p*=0.0235).

### Location of Thermus

*Thermus* was consistently detected in hot water sources. Notably, *Thermus* is a known thermophillic water bacterium and has been isolated previously from hot tap water (Pask-Hughes and Williams, 1975).

**Table 1: Details and differences between manufacture of continental-type spiked cheese trials.**

| **Treatment** | **Control Cheese** | **Experiment 1 Cheese** | **Experiment 2 Cheese** | **Experiment 3 Cheese** |
|---|---|---|---|---|
| Milk Volume | 454 kg | 454 kg | 454 kg | 454 kg |
| Starter Culture (w/v) | | | | |
| *Streptococcus thermophilus* | 500 g | 500 g | 500 g | 250 g |
| *Lactobacillus helveticus* | 250 g | 250 g | 500 g | 500 g |
| *Propionibacterium freudenreichii* | 4 g | 4 g | 4 g | 4 g |
| Test Bacterium cfu ml⁻¹ | | | | |
| *Thermus thermophilus* | 0 | 10⁶ | 10⁶ | 10⁶ |
| Curd Formation | | As Standard | | |
| Cook | | 0.5°C min to 45°C | | |
| | | 1 °C min to 53°C | | |
| Drain pH | | pH 6.30 | | |
| Curd Handling | | Pre-press and mould | | |
| Salting Method | | Brine | | |
| Cheese Size | | 10kg | | |
| Cool Room Ripening | | 8.5°C x 10 days | | |
| Hot Room Ripening | | 22°C x 7 weeks | | |
| Ripening Regime | | 4.5°C after hot room step | | |

**Table 2: Assessment carried out at different stages of manufacture and ripening.**

| **Ripening Time (days)** | **Stages of Ripening** | **Sample Type** | **Microbiological Analysis** | **Compositional Analysis** |
|---|---|---|---|---|
| 0 | Day of manufacture | Milk, Wey, Curd | Tt | pH |
| 1 | After Brining | Cheese | Tt, St, Lh, PAB | pH, Moisture, Salt, Proteins, pH4.6SN, FAA |
| 11 | After 10 days at cool room ripening (8.5°C) | Cheese | Tt, St, Lh, PAB, NSLAB | pH, Moisture, Salt, Proteins, pH4.6SN, FAA |
| 46 | After 5 weeks at warm room ripening (22°C) | Cheese | Tt, St, Lh, PAB, NSLAB | pH, pH4.6SN, FAA, visual examination |
| 60 | End of warm room ripening (22°C) | Cheese | Tt, PAB, NSLAB | pH, pH4.6SN, FAA, visual examination |
| 88 | After 1 month in cold room (4.5 °C) | Cheese | Tt, NSLAB | pH, pH4.6SN, FAA, visual examination |
| 116 | After 2 months in cold room (4.5 °C) | Cheese | Tt, NSLAB | pH, pH4.6SN, FAA, visual examination |
| 144 | After 3 months in cold room (4.5 °C) | Cheese | Tt | pH, pH4.6SN, FAA, visual examination |

Tt - *Thermus thermophilus*; St - *Streptococcus thermophilus*; Lh - *Lactobacillus helveticus*; PAB - Propionic Acid Bacteria; NSLAB - Non-starter lactic acid bacteria; pH4.6SN - pH4.6 s

**Table 3: Composition of cheeses at 11 days post manufacture.**

| | **pH** | **% Moisture** | **% Salt** | **% Protein** |
|---|---|---|---|---|
| **Control** | 5.21 | 41.10 | 1.36 | 24.931 |
| **Exp 1** | 5.24 | 40.80 | 1.25 | 25.271 |
| **Exp 2** | 5.21 | 41.50 | 1.22 | 25.723 |
| **Exp 3** | 5.23 | 40.94 | 1.28 | 24.804 |

| | | | | |
|---|---|---|---|---|
| Data presented in this table are means for three replicate trials. | | | | |

**Table 4: Effect of treatment on colour properties as determined by Hunter L, a, b, dimensions.**

| | | a* value | |
|---|---|---|---|
| Cheese Sample | Area Assessed | 116 d | 144 d |
| Control | Top | -2.22 | -2.22 |
| | Side | -2.20 | -2.17 |
| | Base | -2.01 | -2.32 |
| | Centre | -2.08 | -2.38 |
| | | | |
| Exp 1 | Top | -2.20 | -2.21 |
| | Side | -2.05 | -2.28 |
| | Base | -2.23 | -2.21 |
| | Centre | -1.91 | -1.95 |
| | | | |
| Exp 2 | Top | -2.57 | -2.28 |
| | Side | -2.20 | -2.16 |
| | Base | -2.52 | -2.10 |
| | Centre | -1.75 | -1.34* |
| | | | |
| Exp 3 | Top | -2.08 | -2.14 |
| | Side | -1.91 | -2.35 |
| | Base | -1.75 | -2.13 |
| | Centre | -1.73 | -1.82 |

| | | | |
|---|---|---|---|
| Here we represent a values which indicate formation of redness colour. The results are those taken from 144 d old cheeses * Statistically significant difference compared to control cheese *p*= 0.0009. Data presented in this table are means for three replicate trials. | | | |

### References

ALEGRIA, A., SZCZESNY, P., MAYO, B., BARDOWSKI, J. & KOWALCZYK, M. 2012. Biodiversity in Oscypek, a traditional Polish cheese, determined by culture-dependent and - independent approaches. Applied and Environmental Microbiology, 78, 1890-8.
ALTSCHUL, S. F., GISH, W., MILLER, W., MYERS, E. W. & LIPMAN, D. J. 1990. Basic local alignment search tool. Journal of Molecular Biology, 215, 403-10.
ANDERSEN, C. M., WOLD, J. P. & MORTENSEN, G. 2006. Light-induced changes in semi-hard cheese determined by fluorescence spectroscopy and chemometrics. International Dairy Journal, 16, 1483-1489.
ANDERSSON, A. F., LINDBERG, M., JAKOBSSON, H., BACKHED, F., NYREN, P. & ENGSTRAND, L. 2008. Comparative analysis of human gut microbiota by barcoded pyrosequencing. PloS one, 3, e2836.
BETZOLD, N. 2004. Pink discolouration of Mozzerella cheese. Thesis, University of Wisconsin, Wisconsin, USA.
BOTTAZZI, V., CAPPA, F., SCOLARI, G. & PARISI, M. 2000. Occurring of pink discolouration in Grana cheese made with one-strain starter culture / Comparsa di colorazione rossa in formaggio Grana prodotto con starter monocoltura.. Scienza e Tecnica Lattiero Casearia, 51, 67-74.
BROCK, T. D. & FREEZE, H. 1969. Thermus aquaticus gen. n. and sp. n., a nonsporulating extreme thermophile. Journal of Bacteriology, 98, 289-297.
CAPORASO, J. G., KUCZYNSKI, J., STOMBAUGH, J., BITTINGER, K., BUSHMAN, F. D., COSTELLO, E. K., FIERER, N., PENA, A. G., GOODRICH, J. K., GORDON, J. I., HUTTLEY, G. A., KELLEY, S. T., KNIGHTS, D., KOENIG, J. E., LEY, R. E., LOZUPONE, C. A., MCDONALD, D., MUEGGE, B. D., PIRRUNG, M., REEDER, J., SEVINSKY, J. R., TUMBAUGH, P. J., WALTERS, W. A., WIDMANN, J., YATSUNENKO, T., ZANEVELD, J. & KNIGHT, R. 2010. QIIME allows analysis of high-throughput community sequencing data. Nature Methods, 7, 335-336.
CARINI, S., LODI, R. & GIUSSANI, G. 1979. Pink discoloration in Fontina cheese. Latte, 4,914-920.
CHANG, S.-F., AYRES, J. W. & SANDINE, W. E. 1985. Analysis of cheese for histamine, tyramine, tryptamine, histidine, tyrosine, and tryptophane. Journal of Dairy Science, 68, 2840-2846.
CLAESSON, M. J., WANG, Q., O'SULLIVAN, O., GREENE-DINIZ, R., COLE, J. R., ROSS, R. P. & O'TOOLE, P. W. 2010. Comparison of two next-generation sequencing technologies for resolving highly complex microbiota composition using tandem variable 16S rRNA gene regions. Nucleic Acids Research, 38, e200.
COLE, J. R., WANG, Q., CARDENAS, E., FISH, J., CHAI, B., FARRIS, R. J., KULAM-SYED-MOHIDEEN, A.S., MCGARRELL, D. M., MARSH, T., GARRITY, G. M. & TIEDJE, J. M. 2009. The Ribosomal Database Project: improved alignments and new tools for rRNA analysis. Nucleic Acids Research, 37, D141-5.
DALY, D. F. M., MCSWEENEY, P. L. H. & SHEEHAN, J. 2012. Pink discolouration defect in commercial cheese: a review. Dairy Science & Technology, 92, 439-453.
DOBSON, A., O'SULLIVAN, O., COTTER, P. D., ROSS, P. & HILL, C. 2011. High-throughput sequence-based analysis of the bacterial composition of kefir and an associated kefir grain. FEMS Microbiology Letters, 320, 56-62.
EKMAN, J., KOSONEN, M., JOKELA, S., KOLARI, M., KORHONEN, P. & SALKINOJA-SALONEN, M. 2007. Detection and quantitation of colored deposit-forming Meiothermus spp. in paper industry processes and end products. Journal of Industrial Microbiology & Biotechnology, 34, 203-211.
ERCOLINI, D., DE FILIPPIS, F., LA STORIA, A. & IACONO, M. 2012. "Remake" by high-throughput sequencing of the microbiota involved in the production of water buffalo mozzarella cheese. Applied and Environmental Microbiology, 78, 8142-5.
FENELON, M. A., O'CONNOR, P. & GUINEE, T. P. 2000. The effect of fat content on the microbiology and proteolysis in Cheddar Cheese During Ripening. Journal of Dairy Science, 83,2173-2183.
GIULIANO, G., ROSATI, C. & BRAMLEY, P. M. 2003. To dye or not to dye: biochemistry of annatto unveiled. TRENDS in Biotechnology, 21, 513-516.
GOVINDARAJAN, S. & MORRIS, H. 1973. Pink discoloration in Cheddar cheese. Journal of Food Science, 38, 675-678.
HONG, C., WENDORFF, W. & BRADLEY JR, R. 1995a. Factors affecting light-Induced pink discoloration of annatto-colored cheese. Journal of Food Science, 60, 94-97.
HONG, C., WENDORFF, W. & BRADLEY, R. 1995b. Effects of packaging and lighting on pink discoloration and lipid oxidation of annatto-colored cheeses. Journal of Dairy Science, 78, 1896-1902.
HUSON, D. H., AUCH, A. F., QI, J. & SCHUSTER, S. C. 2007. MEGAN analysis of metagenomic data. Genome Research, 17, 377-386.
IDF 1982. Determination of total solids content (cheese and processed cheese), International Standards 4a Brussels, Belgium: International Dairy Federation.
IDF 1988. Cheese and processed cheese: Determination of chloride content (potentiometric titration method). International Standards 4a Brussels, Belgium: International Dairy Federation.
IDF 1993. Milk: Determination of the nitrogen content (Kjeldahl method) and calculation of crude protein content., International Standards 20b Brussels, Belgium: International Dairy Federation.
KOLARI, M., NUUTINEN, J., RAINEY, F. & SALKINOJA-SALONEN, M. 2003. Colored moderately thermophilic bacteria in paper-machine biofilms. Journal of Industrial Microbiology and Biotechnology, 30, 225-238.
KRUSKAL, W. H. & WALLIS, A. W. 1952. Use of ranks in one-criterion variance analysis. Journal of the American Statistical Association, Vol. 47, pp. 583-621.
MARTLEY, F. G. & MICHEL, V. 2001. Pinkish colouration in Cheddar cheese-description and factors contributing to its formation. Journal of Dairy Research, 68, 327-332.
MASOUD, W., TAKAMIYA, M., VOGENSEN, F. K., LILLEVANG, S., ABU AL-SOUD, W., SORENSEN, S. J. & JAKOBSEN, M. 2011. Characterization of bacterial populations in Danish raw milk cheeses made with different starter cultures by denaturating gradient gel electrophoresis and pyrosequencing. International Dairy Journal, 21, 142-148.
MORTENSEN, G., BERTELSEN, G., MORTENSEN, B. K. & STAPELFELDT, H. 2004. Light-induced changes in packaged cheeses-a review. International Dairy Journal, 14, 85-102.
MORTENSEN, G., SØRENSEN, J. & STAPELFELDT, H. 2002. Light-induced oxidation in semihard cheeses. Evaluation of methods used to determine levels of oxidation. Journal of Agricultural and Food Chemistry, 50, 4364-4370.
PARAMITA, A. & BROOME, M. Pink discolouration in Romano style cheese - role of a-dicarbonyls. 5th IDF Symposium on Cheese Ripening - Cheese 2008, Bern, Switzerland.
PARK, H., REINBOLD, G. & HAMMOND, E. 1967. Role of propionibacteria in split defect of Swiss cheese. Journal of Dairy Science, 50, 820-823.
PASK-HUGHES, R. & WILLIAMS, R. A. 1975. Extremely thermophilic gram-negative bacteria from hot tap water. Journal of General Microbiology, 88, 321-8.
PĚ̌̌̌̌Č̌̌̌̌KOVÁ, M. 1991. Properties of a hyperthermophilic bacterium (Thermus sp.) isolated from a Carlsbad spring. Folia Microbiologica, 36, 515-521.
PELAEZ, C. & NORTHOLT, M. D. 1988. Factors leading to pink discolouration of the surface of Gouda cheese. Netherlands Milk and Dairy Journal, 42, 323-336.
PRUESSE, E., QUAST, C., KNITTEL, K., FUCHS, B. M., LUDWIG, W. G., PEPLIES, J. & GLOCKNER, F. O. 2007. SILVA: a comprehensive online resource for quality checked and aligned ribosomal RNA sequence data compatible with ARB. Nucleic Acids Research, 35, 7188-7196.
QUIGLEY, L., MCCARTHY, R., O'SULLIVAN, O., BERESFORD, T. P., FITZGERALD, G. F., ROSS, R. P., STANTON, C. & COTTER, P. D. 2013. The microbial content of raw and pasteurized cow milk as determined by molecular approaches. Journal of Dairy Science, 96, 4928-4937.
QUIGLEY, L., O'SULLIVAN, O., BERESFORD, T., PAUL ROSS, R., FITZGERALD, G. & COTTER, P. 2012a. A comparison of methods used to extract bacterial DNA from raw milk and raw milk cheese. Journal of Applied Microbiology, 113, 96-105.
QUIGLEY, L., O'SULLIVAN, O., BERESFORD, T. P., ROSS, R. P., FITZGERALD, G. F. & COTTER, P. D. 2011. Molecular approaches to analysing the microbial composition of raw milk and raw milk cheese. International Journal of Food Microbiology, 150, 81-94.
QUIGLEY, L., O'SULLIVAN, O., BERESFORD, T. P., ROSS, R. P., FITZGERALD, G. F. & COTTER, P. D. 2012b. high-throughput sequencing for detection of subpopulations of bacteria not previously associated with artisanal cheeses. Applied and Environmental Microbiology, 78, 5717-5723.
ROESCH, L. F., FULTHORPE, R. R., RIVA, A., CASELLA, G., HADWIN, A. K. M., KENT, A. D., DAROUB, S. H., CAMARGO, F. A. O., FARMERIE, W. G. & TRIPLETT, E. W. 2007. Pyrosequencing enumerates and contrasts soil microbial diversity. The ISME Journal, 1, 283-290.
ROH, S. W., KIM, K. H., NAM, Y. D., CHANG, H. W., PARK, E. J. & BAE, J. W. 2010. Investigation of archaeal and bacterial diversity in fermented seafood using barcoded pyrosequencing. The ISME Journal, 4, 1-16.
SAKAMOTO, N., TANAKA, S., SONOMOTO, K. & NAKAYAMA, J. 2011. 16S rRNA pyrosequencing-based investigation of the bacterial community in Nukadoko, a pickling bed of fermented rice bran. International Journal of Food Microbiology, 144, 352-359.
SHANNON, E. & OLSON, N. 1969. Rapid screening test to predict the tendency of lactic starter cultures to produce pink discoloration in Italian cheese. Journal of Dairy Science, 52, 1678-1680.
SHANNON, E., OLSON, N. & VON ELBE, J. 1968. Pink Discoloration in Italian Varieties of Cheese. Journal of Dairy Science, 51, 613-614.
SHARP, R. J. & WILLIAMS, R. A. 1988. Properties of Thermus ruber strains isolated from Icelandic hot springs and DNA: DNA homology of Thermus ruber and Thermus aquaticus. Applied and Environmental Microbiology, 54, 2049-2053.
SHEEHAN, J. J., FENELON, M. A., WILKINSON, M. G. & MCSWEENEY, P. L. H. 2007. Effect of cook temperature on starter and non-starter lactic acid bacteria viability, cheese composition and ripening indices of a semi-hard cheese manufactured using thermophilic cultures. International Dairy Journal, 17, 704-716.
SHEEHAN, J. J., WILKINSON, M. G. & MCSWEENEY, P. L. 2008. Influence of processing and ripening parameters on starter, non-starter and propionic acid bacteria and on the ripening characteristics of semi-hard cheeses. International Dairy Journal, 18, 905-917.
SHUMAKER, E. & WENDORFF, W. 2007. Factors affecting pink discoloration in annatto-colored pasteurized process cheese. Journal of Food Science, 63, 828-831.
STANDARDS, I. B. 1976. British Standard Methods for Chemical Analysis of Cheese: Determination of pH value., London: British Standards Institute.
TIAN, B. & HUA, Y. 2010. Carotenoid biosynthesis in extremophilic Deinococcus-Thermus bacteria. Trends in microbiology, 18, 512-520.
URICH, T., LANZEN, A., QI, J., HUSON, D. H., SCHLEPER, C. & SCHUSTER, S. C. 2008. Simultaneous assessment of soil microbial community structure and function through analysis of the meta-transcriptome. PloS One, 3, e2527.
WADHWANI, R. & MCMAHON, D. 2012. Color of low-fat cheese influences flavor perception and consumer liking. Journal of Dairy Science, 95, 2336-2346.

## Claims

1. A method of determining the presence in a sample of a source of pink discoloration defect of cheese or a dairy product, comprising the steps of assaying the sample to detect the presence of a *Thermus thermophilus* bacteria in the sample, wherein the sample is an ingredient employed in cheese or dairy product manufacture or is obtained from a dairy product or cheese processing plant, and wherein detection of *Thermus thermophilus* bacteria in the sample indicates that the sample contains a source of pink discoloration defect of cheese or of a dairy product.

2. A method as claimed in Claim 1 in which the sample is obtained from a dairy product processing plant or in which the sample is obtained from a cheese processing plant.

3. A method as claimed in Claim 1 or 2 in which the sample is a swab obtained from a cheese processing plant or an ingredient employed in cheese manufacture.

4. A method as claimed in Claim 2 in which the sample is hot water employed in cheese manufacture.

5. A method as claimed in any preceding Claim in which the sample is assayed using quantitative PCR.

6. A method as claimed in Claim 5 in which the quantitative PCR assay employs a primer pair of SEQUENCE ID NO: 1 and 2.

7. A method of testing a dairy product manufacturing system for a risk of pink discoloration in the dairy product manufactured by the system, in which the dairy product manufacturing system comprises a dairy product manufacturing plant and dairy product manufacturing ingredients processed by the plant, the method comprising the steps of assaying at least one sample obtained from the plant or ingredients to detect the presence of a *Thermus thermophilus* bacteria according to a method of any of Claims 1 to 6, wherein detection of *Thermus thermophilus* bacteria in the at least one sample indicates a risk of pink discoloration in the dairy product manufactured by the system.

8. A method as claimed in Claim 7 in which a plurality of samples are obtained from the plant or ingredients and are selected from milk vat, starter culture vat, pasteuriser, starter culture, hot water, brine, CIP liquid,

9. A method of identifying an origin of pink coloration defect in a dairy product manufacturing system of the type comprising a dairy product manufacturing plant and dairy product manufacturing ingredients processed by the plant, the method comprising the steps of assaying samples obtained from a plurality of different sources selected from locations within the plant or ingredients to detect the presence of a *Thermus thermophilus* bacteria in the samples according to a method of any of Claims 1 to 6, wherein detection of *Thermus thermophilus* bacteria in one of the samples indicates that the origin of the pink coloration defect is the source of the sample.

10. A method as claimed in any one of Claims 7 to 9 in which the dairy product is cheese.

11. A method of modifying a cheese manufacturing system of the type producing cheese having a pink coloration defect, the method comprising the steps of identifying an origin of the pink coloration defect according to a method of Claim 10, and modifying the cheese manufacturing system to remove or treat the origin of the pink coloration defect.

12. A method as claimed in Claim 11 in which the origin of the pink coloration defect is colonisation of a location within the cheese manufacturing plant with *Thermus thermophilus*, wherein the surface is treated to remove the colonisation, or in which the origin of the pink coloration defect is the presence of *Thermus thermophilus* within a cheese manufacturing ingredient , wherein the ingredient is replaced with an ingredient not containing *Thermus thermophilus*

13. A method as claimed in any preceding Claim in which the assay to detect the presence of *Thermus thermophilus* in the sample comprises quantitative PCR, in which the PCR is optionally adapted to detect the presence of an amplicon obtained using a primer pair of SEQ ID NO: 1 and 2.

14. Use of a kit for detecting the presence of a source of pink discoloration defect in a sample, the kit comprising a diagnostic reagent for detecting the presence in the sample of *Thermus thermophilus,* in which the kit comprises a primer pair of SEQUENCE ID NO: 1 or a variant thereof that is specific to *Thermus thermophilus* and SEQUENCE ID NO:2 or a variant thereof that is specific to *Thermus thermophilus.*

## Patentansprüche

1. Verfahren zur Bestimmung der Gegenwart einer Quelle eines rosa Verfärbungsfehlers von Käse oder einem Milchprodukt in einer Probe, das die Schritte der Untersuchung der Probe zum Nachweis der Gegenwart eines *Thermus thermophilus* Bakteriums in der Probe umfasst, wobei die Probe ein Bestandteil ist, der in der Herstellung von Käse oder Milchprodukten eingesetzt wird, oder von einer Verarbeitungsanlage für Milchprodukte oder Käse erhalten wird und wobei der Nachweis von *Thermus thermophilus* Bakterium in der Probe anzeigt, dass die Probe eine Quelle eines rosa Verfärbungsfehlers von Käse oder von einem Milchprodukt enthält.

2. Verfahren nach Anspruch 1, in dem die Probe von einer Verarbeitungsanlage für Milchprodukte erhalten wird oder in dem die Probe von einer Verarbeitungsanlage für Käse erhalten wird.

3. Verfahren nach Anspruch 1 oder 2, in dem die Probe ein Abstrich, der von einer Verarbeitungsanlage für Käse erhalten wird, oder ein Bestandteil ist, der in der Herstellung von Käse eingesetzt wird.

4. Verfahren nach Anspruch 2, in dem die Probe heißes Wasser ist, das in der Herstellung von Käse eingesetzt wird.

5. Verfahren nach einem vorstehenden Anspruch, in dem die Probe unter Verwendung von quantitativer PCR untersucht wird.

6. Verfahren nach Anspruch 5, in dem die quantitative PCR-Untersuchung ein Primer-Paar von SEQUENZ-ID Nr. 1 und 2 einsetzt.

7. Verfahren zum Testen eines Herstellungssystems für Milchprodukte auf ein Risiko von rosa Verfärbung in dem Milchprodukt, das von dem System hergestellt wird, in dem das Herstellungssystem für Milchprodukte eine Herstellungsanlage für Milchprodukte und Herstellungsbestandteile von Milchprodukten, die von der Anlage verarbeitet werden, umfasst,
wobei das Verfahren die Schritte der Untersuchung von mindestens einer Probe, die von der Anlage oder den Bestandteilen erhalten wird, zum Nachweis der Gegenwart eines *Thermus thermophilus* Bakteriums gemäß einem Verfahren nach einem der Ansprüche 1 bis 6 umfasst, wobei der Nachweis des *Thermus thermophilus* Bakteriums in der mindestens einen Probe ein Risiko von rosa Verfärbung in dem Milchprodukt, das von dem System hergestellt wird, anzeigt.

8. Verfahren nach Anspruch 7, in dem eine Vielzahl von Proben von der Anlage oder Bestandteilen erhalten werden und aus Folgenden ausgewählt sind: Milchbehältnis, Starterkulturbehältnis, Pasteurisierer, Starterkultur, heißem Wasser, Salzwasser, CIP-Flüssigkeit.

9. Verfahren zur Identifizierung eines Ursprungs eines rosa Verfärbungsfehlers in einem Herstellungssystem für Milchprodukte der Art, die eine Herstellungsanlage für Milchprodukte und Herstellungsbestandteile von Milchprodukten, die von der Anlage verarbeitet werden, umfasst, wobei das Verfahren die Schritte der Untersuchung von Proben umfasst, die aus einer Vielzahl von unterschiedlichen Quellen erhalten werden, die aus Orten innerhalb der Anlage oder Bestandteilen ausgewählt sind, um die Gegenwart eines *Thermus thermophilus* Bakteriums in den Proben gemäß einem Verfahren nach einem der Ansprüche 1 bis 6 nachzuweisen, wobei der Nachweis des *Thermus thermophilus* Bakteriums in einer der Proben anzeigt, dass der Ursprung des rosa Verfärbungsfehlers die Quelle der Probe ist.

10. Verfahren nach einem der Ansprüche 7 bis 9, in dem das Milchprodukt Käse ist.

11. Verfahren zur Modifizierung eines Herstellungssystems für Käse der Art, die Käse produziert, der einen rosa Verfärbungsfehler aufweist, wobei das Verfahren die Schritte der Identifizierung eines Ursprungs des rosa Verfärbungsfehlers gemäß einem Verfahren nach Anspruch 10 und der Modifizierung des Herstellungssystems für Käse umfasst, um den Ursprung des rosa Verfärbungsfehlers zu entfernen oder zu behandeln.

12. Verfahren nach Anspruch 11, in dem der Ursprung des rosa Verfärbungsfehlers die Besiedlung eines Ortes innerhalb der Herstellungsanlage für Käse mit *Thermus thermophilus* ist, wobei die Oberfläche behandelt wird, um die Besiedlung zu entfernen, oder in dem der Ursprung des rosa Verfärbungsfehlers die Gegenwart von *Thermus thermophilus* innerhalb eines Herstellungsbestandteils von Käse ist, wobei der Bestandteil durch einen Bestandteil ersetzt wird, der kein *Thermus thermophilus* enthält.

13. Verfahren nach einem vorstehenden Anspruch, in dem die Untersuchung zum Nachweis der Gegenwart von *Thermus thermophilus* in der Probe quantitative PCR umfasst, in dem die PCR optional zum Nachweis der Gegenwart eines Amplikons geeignet ist, das unter Verwendung eines Primer-Paars von SEQ.-ID Nr. 1 und 2 erhalten wird.

14. Verwendung eines Kits für den Nachweis der Gegenwart einer Quelle eines rosa Verfärbungsfehlers in einer Probe, wobei der Kit ein diagnostisches Reagenz für den Nachweis der Gegenwart von *Thermus thermophilus* in der Probe umfasst, wobei der Kit ein Primer-Paar von SEQUENZ-ID Nr. 1 oder eine Variante davon, die für *Thermus thermophilus* spezifisch ist, und SEQUENZ-ID Nr. 2 oder eine Variante davon, die für *Thermus thermophilus* spezifisch ist, umfasst.

## Revendications

1. Méthode de détermination de la présence dans un échantillon d'une source de défaut de décoloration rosée d'un fromage ou d'un produit laitier, comprenant les étapes consistant à analyser l'échantillon afin de détecter la présence d'une bactérie de type *Thermus thermophilus* dans l'échantillon, où l'échantillon est un ingrédient employé dans la fabrication de fromages ou de produits laitiers ou est obtenu à partir d'une usine de transformation de fromages ou de produits laitiers, et où la détection d'une bactérie de type *Thermus thermophilus* dans l'échantillon indique que l'échantillon contient une source de défaut de décoloration rosée d'un fromage ou d'un produit laitier.

2. Méthode selon la revendication 1, dans laquelle l'échantillon est obtenu à partir d'une usine de transformation de produits laitiers ou dans laquelle l'échantillon est obtenu à partir d'une usine de transformation de fromages.

3. Méthode selon la revendication 1 ou 2, dans laquelle l'échantillon est un écouvillonnage obtenu à partir d'une usine de transformation de fromages ou d'un ingrédient employé dans la fabrication de fromages.

4. Méthode selon la revendication 2, dans laquelle l'échantillon est de l'eau chaude employée dans la fabrication de fromages.

5. Méthode selon l'une quelconque des revendications précédentes, dans laquelle l'échantillon est analysé par PCR quantitative.

6. Méthode selon la revendication 5, dans laquelle le dosage par PCR quantitative emploie une paire d'amorces de SEQUENCE ID n° 1 et 2.

7. Méthode d'analyse d'un système de fabrication de produits laitiers concernant un risque de décoloration rosée dans le produit laitier fabriqué par le système, dans laquelle le système de fabrication de produits laitiers comprend une usine de fabrication de produits laitiers et des ingrédients de fabrication de produits laitiers transformés par l'usine,
la méthode comprenant l'étape consistant à analyser au moins un échantillon obtenu à partir de l'usine ou des ingrédients afin de détecter la présence d'une bactérie de type *Thermus thermophilus* selon une méthode selon l'une quelconque des revendications 1 à 6, où la détection d'une bactérie de type *Thermus thermophilus* dans le au moins un échantillon indique un risque de décoloration rosée dans le produit laitier fabriqué par le système.

8. Méthode selon la revendication 7, dans laquelle une pluralité d'échantillons sont obtenus à partir de l'usine ou des ingrédients et sont choisis parmi une cuve de lait, une cuve de culture starter, un pasteurisateur, une culture starter, de l'eau chaude, de la saumure, du liquide NEP.

9. Méthode d'identification de l'origine d'un défaut de coloration rosée dans un système de fabrication de produits laitiers du type comprenant une usine de fabrication de produits laitiers et des ingrédients de fabrication de produits laitiers transformés par l'usine, la méthode comprenant les étapes consistant à analyser des échantillons obtenus à partir d'une pluralité de différentes sources choisies parmi des emplacements au sein de l'usine ou des ingrédients afin de détecter la présence d'une bactérie de type *Thermus thermophilus* dans les échantillons selon une méthode selon l'une quelconque des revendications 1 à 6, où la détection d'une bactérie de type *Thermus thermophilus* dans l'un des échantillons indique que l'origine du défaut de coloration rosée est la source de l'échantillon.

10. Méthode selon l'une quelconque des revendications 7 à 9, dans laquelle le produit laitier est constitué de fromage.

11. Méthode de modification d'un système de fabrication de fromage du type produisant du fromage ayant un défaut de coloration rosée, la méthode comprenant les étapes consistant à identifier une origine du défaut de coloration rosée selon une méthode selon la revendication 10, et modifier le système de fabrication de fromage afin d'éliminer ou traiter l'origine du défaut de coloration rosée.

12. Méthode selon la revendication 11, dans laquelle l'origine du défaut de coloration rosée est la colonisation d'un emplacement au sein de l'usine de fabrication de fromage par *Thermus thermophilus,* où la surface est traitée afin d'éliminer la colonisation, ou dans laquelle l'origine du défaut de coloration rosée est la présence de *Thermus thermophilus* au sein d'un ingrédient de fabrication de fromage, où l'ingrédient est remplacé par un ingrédient ne contenant pas *Thermus thermophilus.*

13. Méthode selon l'une quelconque des revendications précédentes, dans laquelle le dosage de détection de la présence de *Thermus thermophilus* dans l'échantillon comprend une PCR quantitative, où la PCR est éventuellement adaptée afin de détecter la présence d'un amplicon obtenu en utilisant une paire d'amorces de SEQ ID n° 1 et 2.

14. Utilisation d'un kit destiné à la détection de la présence d'une source de défaut de décoloration rosée dans un échantillon, le kit comprenant un réactif de diagnostic afin de détecter la présence dans l'échantillon de *Thermus thermophilus,* où le kit comprend une paire d'amorces de SEQUENCE ID n° 1 ou un variant de celle-ci qui est spécifique de *Thermus thermophilus,* et de SEQUENCE ID n° 2 ou un variant de celle-ci qui est spécifique de *Thermus thermophilus.*


### SEQUENCE LISTING

<110> Agriculture and Food Development Authority (TEAGASC)
<120> Detection of a source of pink discoloration defect in a sample
<130> P11409PC00
<150> GB1410948.2
   <151> 2014-06-19
<160> 4
<170> PatentIn version 3.5
<210> 1
   <211> 18
   <212> DNA
   <213> Artificial Sequence
<220>
   <223> Forward primer for Thermus thermophilus polymerase I gene target sequence
<400> 1
   agcctcctcc acgagttc 18
<210> 2
   <211> 20
   <212> DNA
   <213> Artificial Sequence
<220>
   <223> Reverse primer for Thermus thermophilus polymerase I gene target sequence
<400> 2
   gtaggcgagg agcatggggt 20
<210> 3
   <211> 15
   <212> DNA
   <213> Artificial Sequence
<220>
   <223> Forward primer for 16s rRNA
<220>
   <221> misc_feature
   <222> (14)..(14)
   <223> n is a, c, g, or t
<400> 3
   aytgggydta aagng 15
<210> 4
   <211> 20
   <212> DNA
   <213> Artificial Sequence
<220>
   <223> Reverse primer for 16s rRNA
<400> 4
   ccgtcaatty ytttragttt 20